(19) Europäisches Patentamt
European Patent Office
Office européen des brevets

(11) Publication number: **0 101 093**
**B1**

(12) **EUROPEAN PATENT SPECIFICATION**

(45) Date of publication of patent specification: **07.01.88**

(51) Int. Cl.[4]: **C 10 L 1/22**

(21) Application number: **83108081.7**

(22) Date of filing: **16.08.83**

(54) **Diesel fuel composition.**

(30) Priority: **16.08.82 US 408074**
**27.09.82 US 423606**
**27.09.82 US 424054**
**27.09.82 US 424053**
**06.10.82 US 433160**
**15.10.82 US 434632**
**09.11.82 US 440182**

(43) Date of publication of application:
**22.02.84 Bulletin 84/08**

(45) Publication of the grant of the patent:
**07.01.88 Bulletin 88/01**

(84) Designated Contracting States:
**AT BE CH DE FR GB IT LI LU NL SE**

(56) References cited:
**FR-A- 864 878**
**US-A-3 001 857**
**US-A-3 989 476**

(73) Proprietor: **ETHYL CORPORATION**
**Ethyl Tower 451 Florida Boulevard**
**Baton Rouge Louisiana 70801 (US)**

(72) Inventor: **Filbey, Allen Howard**
**13443 Anne Cleeves Avenue**
**Baton Rouge Louisiana 70816 (US)**
Inventor: **Hinkamp, James Benjamin**
**1444 South Bates Street**
**Birmingham Michigan 48009 (US)**
Inventor: **Seemuth, Paul Douglas**
**7623 Oakmount Drive**
**Baton Rouge Louisiana 70816 (US)**
Inventor: **Thomas, Samuel Gabriel, Jr.**
**16459 Chadsford Avenue**
**Baton Rouge Louisiana 70817 (US)**

(74) Representative: **Sandmair, Kurt, Dr. et al**
**Patentanwälte Schwabe, Sandmair, Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 101 093 B1**

Courier Press, Leamington Spa, England.

**Description**

Diesel engines operate by compression ignition. They have compression ratios in the range of 14:1 to 17:1 or higher and for that reason obtain more useful work from a given amount of fuel compared to an Otto cycle engine. Historically, diesel engines have been operated on a petroleum-derived liquid hydrocarbon fuel boiling in the range of about 300—750°F (149—399°C). Recently, because of dwindling petroleum reserves, alcohol and alcohol-hydrocarbon blends have been studied for use as diesel fuel.

One major factor in diesel fuel quality is cetane number. Cetane number is related to ignition delay after the fuel is injected into the combustion chamber. If ignition delays too long, the amount of fuel in the chamber increases and upon ignition results in a rough running engine and increased smoke. A short ignition delay results in smooth engine operation and decreases smoke. Commercial petroleum diesel fuels generally have a cetane number of about 40—55. Alcohols have a much lower cetane value and require the addition of a cetane improver for successful engine operation.

Through the years, many types of additives have been used to raise the cetane number of diesel fuel. These include peroxides, nitrites, nitrates, nitrosocarbamates, and the like. Alkyl nitrates such as amyl nitrate, hexyl nitrate and mixed octyl nitrates have been used commercially with good results.

For example, the FR—PS 864 878 discloses the use of primary aliphatic nitrate esters as ignition quality improving agents. Further, US—PS 3001857 discloses a method of up-grading a low-grade diesel fuel by adding a composite diesel fuel additive consisting essentially of a high-grade hydrocarbon diesel oil and an alkyl nitrate. The composite additive is added to the low-grade diesel oil in an amount from 0,1 to 6% by weight. Moreover, in Menrad et. al. "Alkoholkraftstoffe", page 98, Springer Verlag, September 1982, the use of cyclohexanolnitrate as ignition improving agent is described.

Secondary nitrate esters having nitro substitution are disclosed in U.S. 3,282,983. They are made by reaction of an olefin with dinitrogen tetroxide and oxygen followed by reduction of the intermediate peroxynitrate. According to the disclosure, it is not possible to make a primary nitrate by this method.

British 580,260 and British 586,022, as well as British 604,360, U.S. 3,453,942 and U.S. 2,472,550, are similar and disclose secondary nitrate esters.

The present invention provides an increased cetane rating for a diesel fuel, either hydrocarbon, alcohol or mixed, by the addition of a small but effective amount of an optionally substituted (cyclo)aliphatic nitrate ester.

A preferred embodiment of the invention is a liquid fuel adapted for use in a diesel engine, said fuel being selected from the group consisting of liquid hydrocarbons of the diesel boiling range, alcohols and mixtures thereof, said fuel containing a cetane number increasing amount of fuel soluble 4-morpholine alkanol nitrate. Such compounds contain in their structure the group

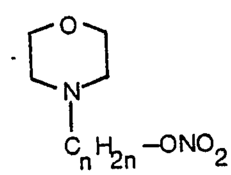

wherein n is an integer from 1 to about 20, more preferably about 1—4. The morpholine heterocyclic ring may be substituted with any of a broad range of substituents as long as they do not render the compound insoluble in diesel fuel.

A still more preferred group of additives have the structure

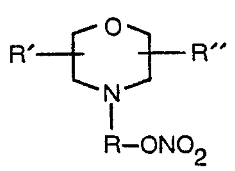

wherein R is a divalent hydrocarbon group containing 1—20 carbon atoms, and R′ and R″ are independently selected from the group consisting of hydrogen, alkyls containing 1—20 carbon atoms, cycloalkyl containing 5—8 carbon atoms, alkenyl containing 2—20 carbon atoms, aryl containing 6—12 carbon atoms, and aralkyl containing 7—12 carbon atoms.

Representative examples of these additives are:

4-(2-methylmorpholine) ethanol nitrate
4-(3-isooctylmorpholine) ethanol nitrate
4-(3-eicosylmorpholine) butanol nitrate
4-(3-cyclopentylmorpholine) hexanol nitrate
4-(2-cyclohexylmorpholine) octanol nitrate
4-(2-cyclooctylmorpholine) dodecanol nitrate
4-(2-propenylmorpholine) propanol nitrate

4-(3-dodecenylmorpholine) ethanol nitrate
4-(3-eicosenylmorpholine) ethanol nitrate
4-(2-phenylmorpholine) ethanol nitrate
4-(3-naphthylmorpholine) butanol nitrate
4-(3-benzylmorpholine) butanol nitrate
4-[2-(alpha-methylbenzyl)morpholine] ethanol nitrate
4-[3-(4-isohexylphenyl)morpholine]ethanol nitrate

and the like.

In a more preferred embodiment, both R′ and R″ in the above structure are hydrogen and R is a divalent hydrocarbon group containing about 1—4 carbon atoms.

These compounds include:

4-morpholine ethanol nitrate
4-morpholine-(2-methylethanol) nitrate
4-morpholine methanol nitrate
4-morpholine butanol nitrate

The most preferred 4-morpholine alkanol nitrate additive is 4-morpholine ethanol nitrate which has the structure

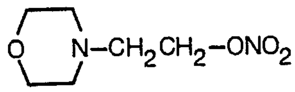

This preferred compound is reported at *Bull. Soc. Chem. 11,* p. 470 (1944).

The additives can readily be prepared by nitration of the corresponding 4-morpholine alkanol compound by standard procedures such as by use of mixed sulfuric-nitric acid or acetic anhydride-nitric acid. The 4-morpholine alkanol is added to the rapidly stirred mixed acid at low temperatures such as −20 to 10°C, more preferably, about −15°C to 0°C.

Example 1

In a reaction vessel was placed 28 ml concentrated sulfuric acid. To this was added 25 g of N-ethanol morpholine to form the ammonium salt. In a second vessel was placed a solution of 14.8 ml concentrated nitric acid and 22 ml concentrated sulfuric acid. The mixed acid was cooled to −14°C. The above N-ethanol morpholine salt was added to this over a two-hour period. The temperature was allowed to rise to the 6.6—7.2°C range. The reaction mixture was stirred an additional 30 minutes and then poured into an ice-water mixture. The aqueous solution was neutralized to pH 8 using sodium carbonate. An upper organic layer formed. The entire mixture was extracted twice with 150 ml portions of diethyl ether. The ether extract was dried over magnesium sulfate and the ether removed under vacuum. The product formed a viscous gel which was soluble in diesel fuel. The product was identified by IR and NMR as 4-morpholine ethanol nitrate.

Other 4-morpholine alkanol nitrate esters can be made by following the above general procedure.

Another preferred embodiment of the invention is liquid fuel adapted for use in a diesel engine, said fuel being selected from the group consisting of liquid hydrocarbons of the diesel boiling range, alcohols and mixtures thereof, said fuel containing a cetane increasing amount of an additive of the formula

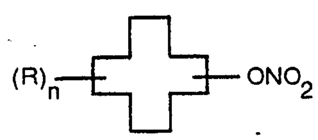

wherein R is selected from the group consisting of hydrogen, alkyl containing 1—12 carbon atoms and alkoxy containing 1—12 carbon atoms and n is an integer from 0 to 3.

Representative examples of these additives are:

4-methyl cyclododecyl nitrate
4-ethyl cyclododecyl nitrate
4-isobutyl cyclododecyl nitrate
4-(2-ethylhexyl) cyclododecyl nitrate
6-n-dodecyl cyclododecyl nitrate
6-methyl cyclododecyl nitrate
4-ethoxy cyclododecyl nitrate
3-isobutoxy cyclododecyl nitrate
3-dodecyloxy cyclododecyl nitrate

The most preferred cyclododecyl nitrate cetane improving additive is the compound cyclododecyl nitrate. This compound is reported at *Chem. Abst., 58,* page 2380d.

The additives can be readily made by nitration of the corresponding cyclododecyl alcohol using a mixed nitric-sulfuric acid or a mixed nitric-acetic anhydride. The following example illustrates the method of making the additives.

Example 2

In a reaction vessel was placed 40 ml acetic acid. This was stirred and cooled to −14°C and then 10.5 ml 90 percent fuming nitric acid was added while keeping the temperature below −7°C. Then, 0.2 g urea was added following which 27.6 g (0.15 mole) of cyclododecanol was added at −14°C to −5°C over a one-hour period. The mixture was stirred 1.5 hours at −5 to −7°C and then poured into an ice-water mixture. The entire aqueous mixture was extracted with diethyl ether and the ether extract was washed with aqueous sodium carbonate and then dried over anhydrous sodium sulfate. The ether was evaporated off at 30°C per 30 mm Hg leaving 33.83 g of white solid identified by IR as predominantly cyclododecyl nitrate.

Other cyclododecyl nitrates can be made following the above general procedure by substituting different cyclododecanols.

The amount of cetane improver added depends on the type of fuel being used, the initial cetane value, and the amount of cetane number increase desired. Alcohol fuels such as methanol, ethanol, isopropanol, isobutanol, hexanol, and the like, have very low cetane values and large amounts of cetane improvers are required. A useful range in which to operate is about 5—25 weight percent cetane improver.

Blends of alcohol and petroleum-derived diesel fuel have higher cetane values and require less cetane improver. A useful range is about 0.5—10 weight percent.

Petroleum-derived distillate fuels in the diesel boiling range require only small amounts of cetane improver to achieve a significant increase in cetane number. Such fuels without any cetane improver generally have cetane numbers in the range of about 25—60. Cetane numbers in the range of 25—35 are considered low and those in the range of 50—60 are considered top grade diesel fuels. Diesel fuels in the 35—50 mid-range are most common. An object of the invention is to upgrade the low cetane number fuels at least into the mid-range and to increase the cetane value of the mid-range fuels into the upper portion of the mid-range (e.g., 45—50) or even into the premium range above 50. It has been found that highly beneficial results can be achieved using as little as 0.05 weight percent of the present additive. Accordingly, a useful concentration range in petroleum derived diesel fuel is about 0.01—5 weight percent and more preferably about 0.05—0.5 weight percent.

The cetane increase caused by the additive was measured in comparison with that caused by a commercial cetane improver, isooctyl nitrate, using a standard cetane engine. The fuel used was a commercial No. 2 petroleum diesel fuel. The results at various concentrations of the improving agent according to the present invention and isooctyl nitrate are shown in the following tables.

The cetane increase caused by the 4-morpholine alkanol nitrate is shown in the following table.

| Concentration | Isooctyl Nitrate | 4-Morpholine ethanol Nitrate |
|---|---|---|
| None | 38.0 | 38.0 |
| 0.05 | 39.3 | 40.07 |
| 0.1 | 40.5 | 41.01 |
| 0.15 | 41.8 | 41.19 |

These results show that the new additives are very effective in raising the cetane number of diesel fuel.

The cetane increase caused by the cyclododecyl nitrate additive was measured using a standard cetane engine in comparison with the increase caused by cyclohexyl nitrate. The fuel was a blend of 28 cetane number light cycle oil and 46 cetane number diesel fuel giving a blend of slightly over 38 cetane number.

| | Cyclohexyl Nitrate | | Cyclododecyl Nitrate | |
|---|---|---|---|---|
| Concentration | CN | Increase | CN | Increase |
| None | 38.33[1] | — | 38.25[2] | — |
| 0.05 Wt. % | 40.02 | 1.69 | 40.77 | 2.52 |
| 0.10 wt. % | 41.47 | 3.14 | 42.02 | 3.77 |
| 0.15 wt. % | 42.69 | 4.36 | 43.13 | 4.88 |

[1] average of 2 runs
[2] average of 2 runs

The increase in cetane number (CN) is measured against the baseline (none). The two baselines are slightly different because, although the fuels were identical, each test series was run on a different day.

The results show that the cyclododecyl nitrate was much more effective compared to the cyclohexyl nitrate. At 0.15 wt. percent, it was 12 percent more effective. At 0.10 wt. percent, it was 20 percent more effective. At 0.05 wt. percent, the cyclododecyl nitrate was a surprising 49 percent more effective than cyclohexyl nitrate.

Other conventional additives may be included in the diesel fuel including antioxidants, pour point depressants, cold flow improvers, cold filter plugging inhibitors, detergents, rust inhibitors and the like, including other cetane improvers.

**Claims**

1. In a liquid fuel adapted for use in a diesel engine, said fuel being selected from liquid hydrocarbons of the diesel boiling range, alcohols and mixtures thereof, containing for the improvement of the cetane number a cetane number increasing amount of an organic nitrate ester, characterized in that said organic nitrate ester is a 4-morpholine alkanol nitrate.

2. A fuel composition as claimed in claim 1 characterized in that said 4-morpholine alkanol nitrate has the structure

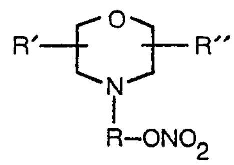

wherein R is a divalent hydrocarbon group containing 1—20 carbon atoms, and R′ and R″ are independently selected from the group consisting of hydrogen, alkyls containing 1—20 carbon atoms, cycloalkyl containing 5—8 carbon atoms, alkenyl containing 2—20 carbon atoms, aryl containing 6—12 carbon atoms, and aralkyl containing 7—12 carbon atoms.

3. A fuel composition as claimed in claim 1 characterized in that said nitrate is 4-morpholine ethanol nitrate.

4. A fuel composition as claimed in claim 1 characterized in that said nitrate ester is a cycloaliphatic nitrate having the structure

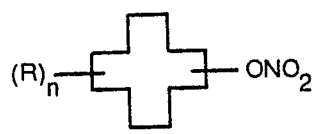

wherein R is selected from hydrogen, alkyl groups containing 1—12 carbon atoms and alkoxy groups containing 1—12 carbon atoms and n is an integer from 0 to 3.

5. A fuel composition as claimed in claim 4 characterized in that said nitrate is cyclododecyl nitrate.

**Patentansprüche**

1. Zur Verwendung in einem Dieselmotor angepaßter flüssiger Kraftstoff ausgewählt aus flüssigen Kohlenwasserstoffen des Dieselsiedebereichs, Alkoholen und Mischungen davon, enthaltend zur Verbesserung der Cetanzahl eine die Cetanzahl erhöhende Menge eines organischen Nitratesters, dadurch gekennzeichnet, daß der organische Nitratester 4-Morpholinalkanolnitrat ist.

2. Kraftstoffzusammensetzung nach Anspruch 1, dadurch gekennzeichnet, daß das 4-Morpholinalkanolnitrat die folgende Struktur aufweist:

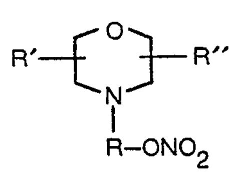

worin R eine divalente Kohlenwasserstoffgruppe mit 1 bis 20 Kohlenstoffatomen bedeutet, R′ und R″ unabhängig ausgewählt sind aus der Gruppe enthaltend Wasserstoff, Alkyl mit 1 bis 20 Kohlenstoffatomen, Cycloalkyl mit 5 bis 8 Kohlenstoffatomen, Alkenyl mit 2 bis 20 Kohlenstoffatomen, Aryl mit 6 bis 12 Kohlenstoffatomen und Aralkyl mit 7 bis 12 Kohlenstoffatomen.

3. Kraftstoffzusammensetzung nach Anspruch 1, dadurch gekennzeichnet, daß das Nitrat 4-Morpholinethanolnitrat ist.

4. Kraftstoffzusammensetzung nach Anspruch 1, dadurch gekennzeichnet, daß der Nitratester ein cycloaliphatisches Nitrat mit der folgenden Struktur ist:

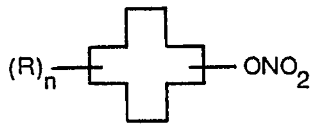

worin R aus Wasserstoff, Alkylgruppen mit 1 bis 12 Kohlenstoffatomen und Alkoxygruppen mit 1 bis 12 Kohlenstoffatomen ausgewählt ist und n eine ganze Zahl von 0 bis 3 bedeutet.

5. Kraftstoffzusammensetzung nach Anspruch 4, dadurch gekennzeichnet, daß das Nitrat Cyclododecylnitrat ist.

**Revendications**

1. Combustible liquide apte à être utilisé dans un moteur Diesel, ledit combustible étant choisi entre des hydrocarbures liquides dans les limites d'ébullition des combustibles Diesel, des alcools et leurs mélanges, contenant, pour l'amélioration de l'indice de cétane, une quantité propre à accroître l'indice de cétane d'un ester du type nitrate organique, caractérisé en ce que l'ester du type nitrate organique est un nitrate de 4-morpholino-alcanol.

2. Composition de combustible suivant la revendication 1, caractérisé en ce que le nitrate de 4-morpholino-alcanol répond à la formule structurale

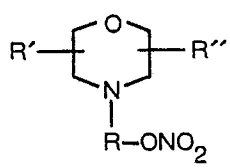

dans laquelle R est un groupe hydrocarboné divalent contenant 1 à 20 atomes de carbone, et R' et R'' sont choisis indépendamment dans le groupe comprenant l'hydrogène, des radicaux alkyle contenant 1 à 20 atomes de carbone, cycloalkyle contenant 5 à 8 atomes de carbone, alcényle contenant 2 à 20 atomes de carbone, aryle contenant 6 à 12 atomes de carbone et aralkyle contenant 7 à 12 atomes de carbone.

3. Composition de combustible suivant la revendication 1, caractérisée en ce que le nitrate est le nitrate de 4-morpholino-éthanol.

4. Composition de combustible suivant la revendication 1, caractérisée en ce que l'ester du type nitrate est un nitrate cycloaliphatique répondant à la formule structurale

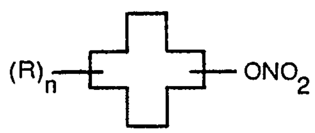

dans laquelle R est choisi entre l'hydrogène, des groupes alkyle contenant 1 à 12 atomes de carbone et des groupes alkoxy contenant 1 à 12 atomes de carbone, et n est un nombre entier de 0 à 3.

5. Composition de combustible suivant la revendication 4, caractérisée en ce que le nitrate est le nitrate de cyclododécyle.